**Europäisches Patentamt**

**European Patent Office**

**Office européen des brevets**

(19)

(11) Veröffentlichungsnummer : **0 383 773 B1**

(12) **EUROPÄISCHE PATENTSCHRIFT**

(45) Veröffentlichungstag der Patentschrift :
**04.03.92 Patentblatt 92/10**

(51) Int. Cl.$^5$ : **A61L 27/00**

(21) Anmeldenummer : **88907304.5**

(22) Anmeldetag : **09.08.88**

(86) Internationale Anmeldenummer :
**PCT/EP88/00711**

(87) Internationale Veröffentlichungsnummer :
**WO 89/01347 23.02.89 Gazette 89/05**

(54) **VERFAHREN ZUR HERSTELLUNG EINES KNOCHENERSATZMATERIALS.**

Verbunden mit 88112916.7/0303941
(europäische
Anmeldenummer/Veröffentlichungsnummer)
durch Entscheidung vom 02.05.91.

(30) Priorität : **19.08.87 DE 3727606**

(43) Veröffentlichungstag der Anmeldung :
**29.08.90 Patentblatt 90/35**

(45) Bekanntmachung des Hinweises auf die
Patenterteilung :
**04.03.92 Patentblatt 92/10**

(84) Benannte Vertragsstaaten :
**AT BE CH DE ES FR GB GR IT LI LU NL SE**

(56) Entgegenhaltungen :
**EP-A- 0 141 004**
**US-A- 4 314 380**

(73) Patentinhaber : **MERCK PATENT
GESELLSCHAFT MIT BESCHRÄNKTER
HAFTUNG
Frankfurter Strasse 250 Postfach 4119
W-6100 Darmstadt (DE)**

(72) Erfinder : **BAUER, Gerd
Lohhofer Strasse 27a
W-8522 Niederndorf (DE)**
Erfinder : **VIZETHUM, Freimut
Mühlgründlein 50
W-8530 Neustadt/Aisch (DE)**

## Beschreibung

Die Erfindung betrifft ein Verfahren zur Herstellung eines Knochenersatzmaterials, bei dem man natürliche Knochen als Ausgangsmaterial verwendet, die Weichteile von dem Knochen entfernt, eine Entfernung der restlichen organischen Substanzen vornimmt und die verbleibende Knochensubstanz anschließend bei Temperaturen zwischen 1100° und 1500° C sintert.

Es ist bekannt, daß Knochenersatzmaterial, das vom Körpergewebe durchwachsen und von diesem Gewebe nicht abgestoßen wird, bevorzugt aus natürlichem Knochenmaterial hergestellt werden kann, aus dem die organischen Substanzen entfernt sind und dessen im wesentlichen unveränderte Matrix anschließend gesintert wird (US-PS 4 314 380; EP-A 141004). Bei diesen bekannten Verfahren wird ein wesentlicher Teil des organischen Materials nach der mechanischen Entfernung von Weichteilen dadurch aus dem Knochenmaterial herausgelöst, daß der Knochen mit einer Wasserstoffperoxid-Lösung behandelt wird. Abgesehen davon, daß eine derartige Behandlung umständlich ist und wegen der bleichenden Wirkung einer solchen Lösung Vorsichtsmaßnahmen erfordert, gelingt es auf diese Weise nicht vollständig, das organische Material aus dem Knochengerüst zu entfernen. Bei dem anschließenden Brennen des Knochens bei erhöhten Temperaturen verbrennt das restliche organische Material, und dabei können flüchtige Bestandteile in solchen Mengen auftreten, daß dadurch die Feinstruktur der Knochenmatrix beschädigt wird. Dabei ist zu berücksichtigen, daß die Knochenmatrix ihre mechanische Festigkeit erst beim Sintern erhält, vor dem Sintern hingegen weist die Matrix nur eine sehr geringe mechanische Stabilität auf.

Es ist Aufgabe der Erfindung, ein gattungsgemäßes Verfahren derart zu verbessern, daß bei einer Vereinfachung des Herstellungsverfahrens gleichzeitig eine schonendere Behandlung des Knochenmaterials und eine weitergehende Beibehaltung der Struktur des Knochenmaterials ermöglicht wird.

Diese Aufgabe wird bei einem Verfahren der eingangs beschriebenen Art erfindungsgemäß dadurch gelöst, daß man den Knochen nach der Entfernung der Weichteile bei einer erhöhten Temperatur bis maximal 150° C trocknet, daran anschließend bei Luftunterschuß oder unter reduzierender Atmosphäre die Temperatur auf etwa 550° C erhöht, in einem nächsten Schritt den Knochen bei Luftüberschuß auf eine Temperatur zwischen 750° und 800° C bringt und daß man daran die Sinterung anschließt.

Dadurch, daß man das Knochenmaterial nach der schonenden Trocknung bei maximal 150° C bei Luftunterschuß oder unter reduzierender Atmosphäre einer erhöhten Temperatur unterzieht, stellt sich eine Pyrolyse ein, also eine thermische Zersetzung des organischen Materials, welche zu einer Verkohlung des organischen Knochenmaterials unter Abspaltung flüchtiger Komponenten führt.

Im nächsten Schritt wird dann bei Luftüberschuß bei Temperaturen bis maximal 800° C die Kohle verbrannt. Leicht flüchtige Komponenten sind bei diesem reinen Verbrennungsvorgang nicht mehr vorhanden, so daß eine Beschädigung der anorganischen Knochenmatrix vermieden wird.

Das beschriebene Verfahren hat den Vorteil, daß keinerlei chemische Behandlung des Knochens notwendig ist, sondern daß nach der mechanischen Entfernung der Weichteile der Knochen ausschließlich einer Wärmebehandlung unterzogen wird, die in verschiedene Abschnitte unterteilt ist.

Um die Trocknung möglichst schonend durchzuführen, kann vorgesehen sein, daß man den Knochen zwischen 24 und 72 Sturiden bei einer erhöhten Temperatur von maximal 150° C trocknet. Außerdem ist es günstig, wenn man bei der Trocknung die Temperatur allmählich von Umgebungstemperatur auf die Maximaltemperatur von 150° C erhöht. Allmählich bedeutet dabei, daß die Erhöhung kontinuierlich über den gesamten Trocknungszeitraum erfolgen kann. Es wird dadurch vermieden, daß bei der Siedetemperatur des Wassers eine schlagartige Verdampfung einsetzt, die das Knochenmaterial beschädigen könnte. Durch die allmähliche Erhöhung der Temperatur erhält man ein stetiges Verdampfen der leicht flüchtigen Komponenten über den gesamten Trocknungszeitraum, wobei keine größeren Gasentwicklungen auftreten.

Es kann weiterhin vorgesehen sein, daß man den Knochen etwa 8 bis 24 Stunden unter Luftunterschuß oder unter reduzierender Atmosphäre behandelt. Dabei wird die Temperatur vorzugsweise allmählich auf 550° C erhöht, wobei auch hier allmählich bedeutet, daß die Temperatur über den gesamten Behandlungszeitraum stetig erhöht wird. Auch diese Maßnahme trägt dazu bei, daß Gaskomponenten unterschiedlicher Flüchtigkeit nacheinander gebildet werden, so daß zu keinem Zeitpunkt unerwünscht hohe Gasentwicklungen auftreten.

Das Brennen des Knochens bei Luftüberschuß bei einer Temperatur von 750° und 800° C wird vorzugsweise 4 bis 24 Stunden lang durchgeführt, wobei es auch hier günstig sein kann, bei dem Brennen des Knochens die Temperatur allmählich auf etwa 800° C zu steigern.

Die Sinterung wird vorzugsweise 2 bis 20 Stunden lang bei Maximaltemperaturen von 1100 bis 1400° C durchgeführt, die Abkühlung erfolgt langsam, beispielsweise über einen Zeitraum von 10 bis 20 Stunden. Es hat sich als besonders vorteilhaft herausgestellt, wenn man nach dem Sintern den Knochen beim Abkühlen über einen Zeitraum von 2 bis 6 Stunden bei einer Temperatur zwischen 1120° und 1160° C hält und das Knochenmaterial danach mit Ofencharakteristik abkühlt. Man erhält dadurch eine Erhöhung der mechanischen Festigkeit, da kleine Mengen an Zweitphase zur Aus- und Umkristallisation gebracht werden können.

Nachfolgend werden einige Beispiele für die Durchführung des erfindungsgemäßen Verfahrens angegeben:

Beispiel 1:

Tierknochen wird durch Auskochen teilweise von seinen organischen Bestandteilen befreit. Anhaftende Weichteile werden mechanisch entfernt.

Die gewünschte Formgebung erfolgt z.B. durch Zerkleinerung mittels Säge.

Daran schließt sich eine Trocknung mit kontinuierlicher Temperatursteigerung von Raumtemperatur bis 150° C im Verlauf von 26 h an.

Es folgt eine Pyrolyse der organischen Bestandteile durch Temperatursteigerung z.B. mit 25 K/h unter Luftunterschuß.

Danach wird das Material z.B. mit 25 K/h im Luftüberschuß auf 800° C gebracht. Der anschließende Sinterprozeß wird von Raumtemperatur bis z.B. 1450° C mit einer Aufheizrate von 100 K/h und einer Haltezeit von 10 Stunden durchgeführt. Die Abkühlung erfolgt mit ebenfalls mit 100 K/h.

Beispiel 2:

Die Schritte der Vorbereitung und der Pyrolyse mit anschliessender Ausbrennung wie im Beispiel 1, die Sinterung erfolgt mit einer Aufheizrate von 100 K/h auf 1250° und einer Haltezeit von 15 Stunden. Während des Abkühlvorganges mit Ofencharakteristik wird bei 1120° eine Haltezeit von 4 Stunden eingelegt.

Es hat sich herausgestellt, daß die Mineralphase des Knochens vollständig erhalten bleibt, wenn die maximale Sintertemperatur bei 1400° C oder darunter liegt. Bei höheren maximalen Sintertemperaturen beginnt eine thermische Zersetzung der Mineralphase, die im Organismus zu einer Erhöhung der Resorption führt. Auf Diese Weise ist es möglich, gezielt die Zusammensetzung der Knochenkeramik so zu verschieben, daß eine mehr oder weniger schnelle Resorption erzwungen wird. Dies ist für manche Anwendungen erwünscht, z.B. bei der Therapie von Osteomyelitis.

**Patentansprüche**

1. Verfahren zur Herstellung eines Knochenersatzmaterials, bei dem man natürliche Knochen als Ausgangsmaterial verwendet, die Weichteile von dem Knochen entfernt, eine Entfernung der restlichen Substanzen vornimmt und die verbleibende Knochensubstanz anschließend bei Temperaturen zwischen 1100° und 1500° C sintert, dadurch gekennzeichnet, daß man den Knochen nach der Entfernung der Weichteile bei einer erhöhten Temperatur bis maximal 150° C trocknet, daran anschließend bei Luftunterschuß oder unter reduzierender Atmosphäre die Temperatur auf etwa 550° C erhöht, in einem nächsten Schritt den Knochen bei Luftüberschuß auf eine Temperatur zwischen 750° und 800° C bringt, und daß man daran die Sinterung anschließt.

2. Verfahren nach Anspruch 1, dadurch gekennzeichnet, daß man den Knochen zwischen 24 und 72 Stunden bei einer erhöhten Temperatur von maximal 150° C trocknet.

3. Verfahren nach Anspruch 1 oder 2, dadurch gekennzeichnet, daß man bei der Trocknung die Temperatur allmählich von Umgebungstemperatur auf die Maximaltemperatur 150° C erhöht.

4. Verfahren nach einem der voranstehenden Ansprüche, dadurch gekennzeichnet, daß man den Knochen etwa 8 bis 24 Stunden unter Luftunterschuß oder unter reduzierender Atmosphäre behandelt.

5. Verfahren nach einem der voranstehenden Ansprüche, dadurch gekennzeichnet, daß man die Temperatur während der Behandlung des Knochens unter Luftunterschuß oder einer reduzierenden Atmosphäre allmählich auf 550° C erhöht.

6. Verfahren nach einem der voranstehenden Ansprüche, dadurch gekenzeichnet, daß man den Knochen etwa 4 bis 24 Stunden bei Lüftüberschuß auf einer Temperatur von maximal 750° bis 800° C hält.

7. Verfahren nach einem der voranstehenden Ansprüche, dadurch gekennzeichnet, daß man bei dem Brennen des Knochens unter Luftüberschuß die Temperatur allmählich bis auf etwa 800° C steigert.

8. Verfahren nach einem der voranstehenden Ansprüche, dadurch gekennzeichnet, daß die Sinterung 2 bis 20 Stunden bei Maximaltemperaturen von 1100 bis 1400° C durchgeführt wird.

9. Verfahren nach einem der voranstehenden Ansprüche, dadurch gekennzeichnet, daß die Temperatur beim Sintern allmählich auf die maximale Sintertemperatur erhöht wird.

10. Verfahren nach einem der voranstehenden Ansprüche, dadurch gekennzeichnet, daß nach dem Sintern der Knochen beim Abkühlen über einen Zeitraum von 2 bis 6 Stunden bei einer Temperatur zwischen 1120° bis 1160° C gehalten wird.

## Claims

1. Process for producing a bone replacement material in which natural bones are used as starting material, the soft tissues are removed from the bone, a removal of the residual substances is effected and the remaining osseous substance is then sintered at temperatures between 1100° and 1500°C, characterized in that the bone is dried at an elevated temperature of up to a maximum of 150°C after the removal of the soft tissues, the temperature is then raised with a reduced air supply or in a reducing atmosphere to about 550°C, in a following step the bone is heated to a temperature of between 750° and 800°C with an increased air supply, and in that sintering takes place subsequently.

2. Process according to claim 1, characterized in that the bone is dried at an elevated temperature of up to a maximum of 150°C for between 24 and 72 hours.

3. Process according to claim 1 or 2, characterized in that, during drying, the temperature is gradually increased from ambient temperature to the maximum temperature of 150°C.

4. Process according to one of the preceding claims, characterized in that the bone is treated for approximately 8 to 24 hours with a reduced air supply or in a reducing atmosphere.

5. Process according to one of the preceding claims, characterized in that the temperature is gradually increased to 550°C during the treatment of the bone with a reduced air supply or in a reducing atmosphere.

6. Process according to one of the preceding claims, characterized in that the bone is kept at a maximum temperature of 750° to 800°C for approximately 4 to 24 hours with an increased air supply.

7. Process according to one of the preceding claims, characterized in that the temperature is gradually increased to approximately 800°C when incinerating the bone with an increased air supply.

8. Process according to one of the preceding claims, characterized in that the sintering is carried out for 2 to 20 hours at maximum temperatures of 1100 to 1400°C.

9. Process according to one of the preceding claims, characterized in that the temperature during sintering is gradually increased to the maximum temperature of sintering.

10. Process according to one of the preceding claims, characterized in that, after sintering, the bone is, during cooling, kept at a temperature of between 1120° to 1160°C for a period of 2 to 6 hours.


## Revendications

1. Procédé de préparation d'un matériau de remplacement des os dans lequel on utilise comme matière première des os naturels, on en élimine les parties molles, on en élimine les matières restantes puis on fritte la matière osseuse résiduelle à des températures de 1100 à 1500°C, ce procédé se caractérisant en ce que, après élimination des parties molles, on sèche les os à une température maximale de 150°C puis on porte la température à 550°C environ dans un défaut d'air ou en atmosphère réductrice, on porte les os dans un stade opératoire subséquent à une température de 750 à 800°C dans un excès d'air nuis on procède au frittage.

2. Procédé selon la revendication 1, caractérisé en ce que l'on sèche les os pendant 24 à 72 h à une température de 150°C au maximum.

3. Procédé selon la revendication 1 ou 2, caractérisé en ce que, au séchage, on augmente peu à peu la température depuis la température ambiante jusqu'à la température maximale de 150°C.

4. Procédé selon l'une des revendications qui précèdent, caractérisé en ce que l'on traite les os pendant 8 à 24 h environ dans un défaut d'air ou en atmosphère réductrice.

5. Procédé selon l'une des revendications qui précèdent, caractérisé en ce que, au cours du traitement des os dans un défaut d'air ou en atmosphère réductrice, on porte peu à peu la température à 550°C.

6. Procédé selon l'une des revendications qui précèdent, caractérisé en ce que l'on maintient les os pendant 4 à 24 h environ dans un excès d'air à une température maximale de 750 à 800°C.

7. Procédé selon l'une des revendications qui précèdent, caractérisé en ce que, à la combustion des os dans un excès d'air, on porte peu à peu la température à 800°C environ.

8. Procédé selon l'une des revendications qui précèdent, caractérisé en ce que l'on fritte pendant 2 à 20 h à des températures maximales de 1100C à 1400°C.

9. Procédé selon l'une des revendications qui précèdent, caractérisé en ce que, au frittage, la température est portée peu à peu jusqu'à la température maximale de frittage.

10. Procédé selon l'une des revendications qui précèdent, caractérisé en ce que, après le frittage et au cours du refroidissement, les os sont maintenus pendant une durée de 2 à 6 h à une température de 1120 à 1160°C.